# EUROPÄISCHE PATENTSCHRIFT

(11) **EP 3 088 510 B1**
(45) Veröffentlichungstag und Bekanntmachung des Hinweises auf die Patenterteilung: **08.09.2021**
(21) Anmeldenummer: 16167128.4
(22) Anmeldetag: 26.04.2016
(51) Int. Cl.: C12M 1/107, C12M 1/00, C12P 5/02, C12M 1/12

(54) **VERFAHREN ZUR AUFBEREITUNG VON GÄRRESTEN AUS BIOGASANLAGEN SOWIE VORRICHTUNG ZUR DURCHFÜHRUNG DES VERFAHRENS**
METHOD FOR PROCESSING FERMENTATION RESIDUES FROM BIOGAS FACILITIES AND DEVICE FOR CARRYING OUT THE METHOD
PROCÉDÉ DE TRAITEMENT DE RÉSIDUS DE FERMENTATION PROVENANT D'INSTALLATION DE BIOGAZ ET DISPOSITIF D'EXÉCUTION DU PROCÉDÉ

(30) Priorität: 28.04.2015 DE 102015106497
(43) Veröffentlichungstag der Anmeldung: 02.11.2016
(73) Patentinhaber: Bio Technologies Patente GmbH, 44379 Dortmund (DE)
(72) Erfinder: Hölper, Krystian, 44269 Dortmund (DE)
(74) Vertreter: Meinke, Jochen

(56) Entgegenhaltungen:
- EP-A1- 2 698 424
- WO-A1-2008/040038
- WO-A1-2014/032798
- DE-A1- 3 025 144
- DE-A1- 19 733 813
- DE-A1-102005 012 936
- DE-A1-102010 005 253
- DE-A1-102010 033 442
- DE-A1-102012 004 497
- DE-A1-102012 212 298
- DE-A1-102013 209 734
- Liquids to Value - GEA: "Gärresteaufbereitung in Biogasanlagen mit Westfalia Separator aquaflow", , 19 March 2018 (2018-03-19), 59302 Oelde (DE) Retrieved from the Internet: URL:http://www.pitt-gmbh.de/downloads/biog as_de.pdf [retrieved on 2021-03-05]

## Beschreibung

Die Erfindung betrifft ein Verfahren zur Aufbereitung von Gärresten aus Biogasanlagen, bei welchem die Flüssigphase der Gärreste einer Flotationseinrichtung zugeführt wird. Ferner betrifft die Erfindung eine Vorrichtung zur Durchführung eines solchen Verfahrens.

Zur Aufbereitung von Gärresten aus Biogasanlagen sind unterschiedliche Systeme bzw. Verfahren bekannt. Den meisten Verfahren dieser Art ist gemeinsam, dass die Gärreste zunächst mechanisch in eine Feststoff- und eine Flüssigphase getrennt werden, dieser Vorgang erfolgt in einer geeigneten Separatoreinrichtung. Nach dieser Fest-/Flüssig-Trennung verbleibt üblicherweise eine Flüssigphase, die rund 80 % des Gesamtstoffstromes der Gärreste ausmacht. Diese Flüssigphase wird je nach eingesetztem Verfahren unterschiedlich weiter aufbereitet.

WO 2008/040038 offenbart eine Vorrichtung zur Durchführung des Verfahrens zur Aufbereitung von Gärresten.

Aus DE 10 2012 212 298 A1 ist es bekannt, die Flüssigphase einer Verdampfereinrichtung zum Aufteilen der Flüssigphase in Wasser, eine erste Düngephase und Feststoffrückstände zuzuführen. Eine solche Verdampfereinrichtung ist jedoch sehr energieaufwendig.

Aus der Broschüre "Gärrestaufbereitung in Biogasanlagen mit Westfalia Separator® aquaflow", die unter http://www.pitt-gmbh.de/downloads/biogas_de.pdf erhältlich ist, ist ein Verfahren zur Aufbereitung von Gärresten bekannt, bei welchem die Flüssigphase in einer Flotationseinrichtung weiter aufbereitet wird. Die Flotationseinrichtung hat die Aufgabe, suspendierte oder kolloidale Stoffe mit Hilfe von Gasblasen aus der Flüssigphase zu separieren. Dazu wird die Flüssigphase komprimiert und durch eine Druckluftatmosphäre geführt. Die aus der Flotationseinrichtung entnommene Flüssigphase kann anschließend noch weiter behandelt werden, z.B. durch membrantechnische, biologische oder chemische Verfahren.

Aus EP 1 294 474 B2 sind ein Verfahren und eine Vorrichtung zur physikalischchemischen Behandlung fluider Medien bekannt, die ebenfalls als Flotationseinrichtung verwendet werden kann.

Aufgabe der Erfindung ist es, eine Lösung anzugeben, mit der die Aufbereitung der aus der Flotationseinrichtung entnommenen Flüssigphase weiter optimiert werden kann.

Diese Aufgabe wird mit einem Verfahren gemäß Anspruch 1 erfindungsgemäß dadurch gelöst, dass die aus der Flotationseinrichtung entnommene Flüssigphase in einen temperierten Behälter eingeleitet und entspannt wird und in diesem über eine Verweildauer von mehreren Stunden verbleibt, wobei der Behälter über seiner Höhe mit Aufwuchskörpern befüllt ist und wobei frei gesetztes oder entstehendes Gas am Behälterkopf abgeführt wird.

Die Flüssigphase, welche einem Flotationsverfahren unter Überdruck (z.B. 5 bar), beispielsweise in einer Flotationseinrichtung gemäß EP 1 294 474 B2 unterzogen worden ist, wird unter schneller Entspannung in den temperierten Behälter eingeleitet, wodurch eine schnelle Entgasung erfolgt. Die Flüssigphase weist nach dem Flotationsverfahren unter anderem feinste biologische Partikel auf, die durch die besonderen physikalischen Scher- und Gravitationskräfte beim Flotationsverfahren aufgeschlossen worden sind. Die darin noch vorhandenen schnell verfügbaren biologischen Kohlenstoffe werden erfindungsgemäß im Behälter gemeinsam mit den noch verfügbaren Säuren abgebaut, wobei der schnelle Abbau über mehrere Stunden durch die Temperierung des Behälters ermöglicht und durch die Aufwuchskörper begünstigt wird, welche als Siedlungsfläche für methanbildende Mikroorganismen dienen. Bei dem Prozess frei werdende oder entstehende Gase werden am Behälterkopf abgeführt und können z.B. energetisch genutzt werden. Als flüssiges Endprodukt aus dem Behälter entsteht stark entlastetes Flüssigmaterial, das einem Endlager zugeführt werden kann.

Je nach Art und Zusammensetzung der Gärreste sind die Verfahrensparameter im Behälter geeignet zu wählen. Als besonders bevorzugt hat sich herausgestellt, dass die Temperatur im Behälter zwischen 35° und 60° C, vorzugsweise 52,5° C, beträgt. Die Verweildauer der Flüssigphase im Behälter beträgt vorzugsweise zwischen 2 und 48 Stunden, als besonders bevorzugt hat sich eine Verweildauer von etwa 12 Stunden herausgestellt. Die Verweildauer ist selbstverständlich von der Art der Gärreste abhängig.

Die Erfindung sieht auch eine Vorrichtung gemäß Anspruch 4 zur Durchführung des vorbeschriebenen Verfahrens vor, welche einen temperierten Behälter mit einem Zulauf für die Flüssigphase, einen Gasaustritt am Behälterkopf, einen Bioschlammaustritt am Behälterboden und einen Austritt für die Flüssigphase aufweist, wobei der Behälter über seiner Höhe mit Aufwuchskörpern befüllt ist.

Damit die Aufwuchskörper bei der Produktentnahme aus dem Behälter im Behälter verbleiben, ist vorgesehen, dass zwischen dem Austritt für die Flüssigphase und dem mit Aufwuchskörpern befüllen Bereich des Behälters eine für die Aufwuchskörper undurchlässige Trennwand angeordnet ist. Diese kann je nach Behälterform unterschiedlich gestaltet sein. Wenn der Behälter zylindrisch ausgebildet ist, kann die Trennwand z.B. querschnittlich halbkreisförmig ausgebildet sein. Wenn der Behälter aufgrund bestehender Sicherheitsvorschriften doppelwandig ausgebildet sein muss, können Leitungsanschlüsse nur im Bereich des Behälterkopfes vorgesehen werden. In diesem Falle ist deshalb bevorzugt vorgesehen, dass der Zulauf im Bereich des Behälterkopfes angeordnet ist und in eine Beregnungseinrichtung mündet. Die aus der Flotationseinrichtung entnommene Flüssigphase entspannt dabei in der Beregnungseinrichtung, wodurch der Gasaustritt begünstigt wird und außerdem eine bessere Schichtenbildung im Behälter erreicht wird.

Ferner ist vorteilhaft vorgesehen, dass der Austritt für die Flüssigphase und der Bioschlammaustritt an Austrittsleitungen angeschlossen sind, welcher am Behälterkopf aus dem Behälter nach außen geführt sind.

Weiterhin ist vorteilhaft vorgesehen, dass oberhalb der Aufwuchskörper im Behälter wenigstens ein Schaumsieb angeordnet ist, um zu gewährleisten, dass am Kopf des Behälters nur Gas ohne Flüssigpartikel austritt.

Im Bereich des Gasaustrittes ist vorzugsweise eine Stoffaustauschpackung angeordnet, die in unterschiedlichen Ausgestaltungen bekannt ist. So sind Hochleistungsfüllkörper für die chemische und physikalische Absorption, Desorption und für Biowäscher geeignet.

Schließlich ist bevorzugt vorgesehen, dass der Behälter wenigstens einen weiteren Entnahmeanschluss aufweist. Der oder die weiteren Entnahmeanschlüsse in unterschiedlicher Höhenlage am Behälter dienen dazu, unterschiedlich konzentrierte Flüssigphasen aus dem Behälter entnehmen zu können, die ggf. noch weiter unterschiedlich nachbehandelt werden können, z.B. durch Ultraschallbehandlung, UV-Bestrahlung oder Belüftung in offenen Behältern zur Nitrifikation.

Der Behälter kann grundsätzlich unterschiedliche geometrische Formen und Abmessungen aufweisen. Vorzugsweise weist der Behälter einen zylindrischen Querschnitt und ein Behältervolumen von 10 bis 60 m³ auf. Bei einem Volumen von z.B. 18 m³ kann der Behälter z.B. einen Durchmesser von 2 m und eine Höhe von ca. 6 m aufweisen, bei einem Durchmesser von 2,8 m dagegen nur eine Höhe von 3 m. Bevorzugt sind jedoch Behälter mit einer möglichst großen Höhe, um das Absetzverhalten des Bioschlammes zu begünstigen, damit dieser abgesaugt werden kann. Ferner ergeben sich bei hohen Behältern besser getrennte Schichten im Gärrest, im Behälter, die den Behälter getrennt entnommen und unterschiedlichen Verwertungen zugeführt werden können.

Die Erfindung ist nachstehend anhand der Zeichnung beispielhaft näher erläutert. Diese zeigt in
- Fig. 1: eine schematische Seitenansicht eines Behälters nach einer ersten Ausführungsform,
- Fig. 2: einen Querschnitt durch den Behälter nach Fig. 1,
- Fig. 3: eine schematische Seitenansicht eines Behälters nach einer zweiten Ausführungsform und
- Fig. 4: einen Querschnitt durch den Behälter nach Fig. 3.

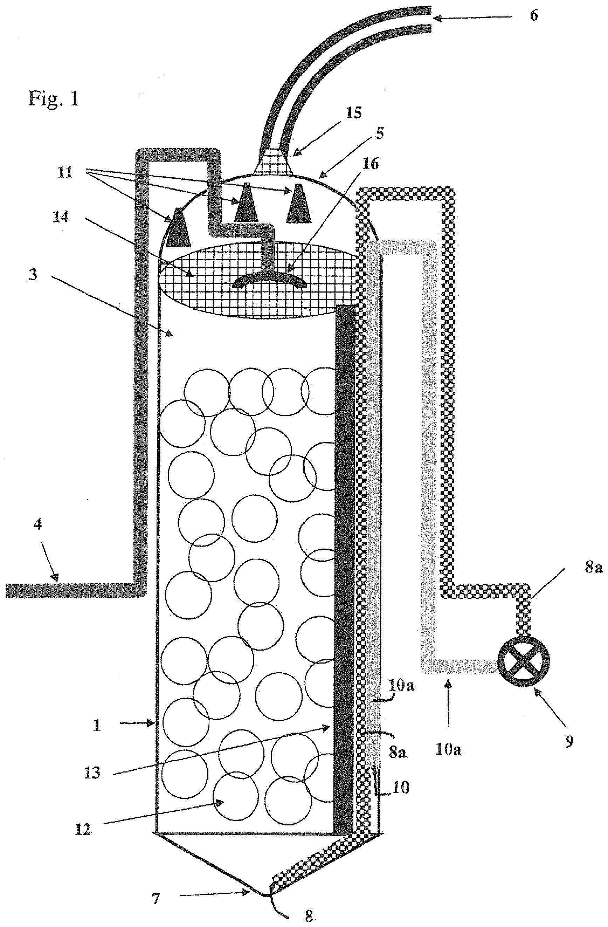

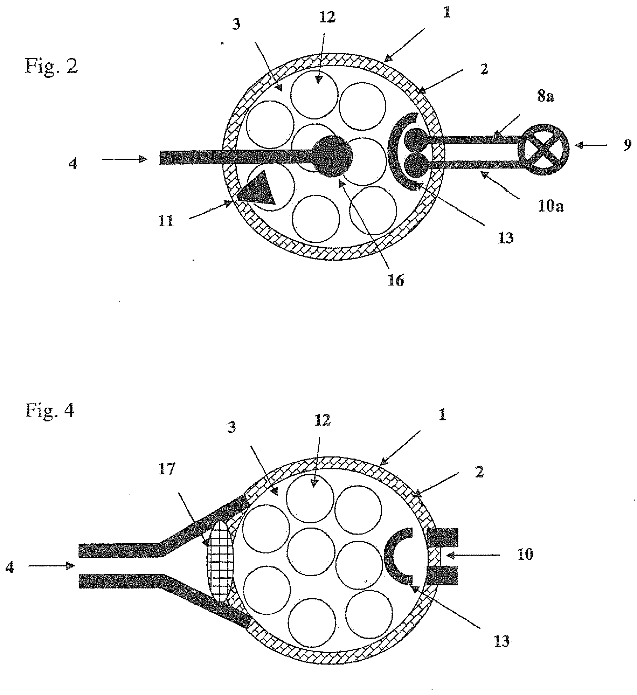

Eine Vorrichtung zur Aufbereitung von Gärresten aus Biogasanlagen weist einen Behälter 1 auf, der, wie im Ausführungsbeispiel dargestellt, vorzugsweise einen zylindrischen Querschnitt und ein Behältervolumen von z.B. 18 m³ aufweist. Der Behälter 1 ist zur Temperierung mit einer Heizung 2 in der Behälterwandung oder einer Durchflussheizung im Zulauf 4 versehen, die auf unterschiedliche Weise ausgeführt sein kann, aber so dimensioniert ist, dass im Innenraum 3 des Behälters 1 ein Temperaturniveau von 35 bis 60° C erzeugt werden kann. Beim Ausführungsbeispiel nach Fig. 1 und 2 ist der Behälter doppelwandig ausgebildet, was nicht näher dargestellt ist.

Der Behälter 1 weist einen Zulauf 4 für eine Flüssigphase auf, der im Bereich des Behälterkopfes 5 angeordnet ist. Dabei erweitert sich der Zulauf 4 zum Behälter 1 hin, was eine Entspannung der Flüssigphase zwecks Entgasung begünstigt. Diese Erweiterung erfolgt dadurch, dass der Zulauf 4 in einer Beregnungseinrichtung 16 mündet. Diesem Zulauf 4 ist eine nicht dargestellte Flotationseinrichtung vorgeschaltet, welche beispielsweise so ausgebildet sein kann, wie die in der EP 1 294 474 B2 beschriebene Vorrichtung. Dieser Flotationseinrichtung wird die Flüssigphase von Gärresten aus Biogasanlagen zugeführt, welche z.B. durch eine mechanische Vortrennung der Gärreste erzeugt bzw. generiert worden ist.

Der Behälter 1 weist am Behälterkopf 5 einen Gasaustritt 6 auf und am Behälterboden 7 einen Bioschlammaustritt 8 auf. Ferner weist der Behälter 1 ein Austritt 10 für die aufbereitete Flüssigphase auf. Über der Höhe des Behälters 1 können weitere Entnahmeanschlüsse 11 und/oder Schaugläser angeordnet sein.

Der Behälterinnenraum 3 ist über seiner gesamten Höhe mit Aufwuchskörpern 12 befüllt. Diese Aufwuchskörper bestehen z.B. aus gesintertem Kunststoffmaterial. Die Aufwuchskörper 12 sind durch eine Trennwand 13 vom Austritt 10 getrennt. Dabei ist die Trennwand 13 so gestaltet bzw. dimensioniert und angeordnet, dass die Aufwuchskörper 12 nicht in den Bereich des Austritts 10 gelangen können. Die Trennwand kann, wie dies Fig. 2 zeigt, z.B. einen halbkreisförmigen Querschnitt aufweisen. Aufgrund der doppelwandigen Ausführung des Behälters 1 dürften die Leitungsanschlüsse nur über den Behälterkopf 5 erfolgen. Deshalb ist der Austritt 10 für die Flüssigphase an eine Austrittsleitung 10a und der Bioschlammaustritt 8 an eine Austrittsleitung 8a angeschlossen, welche beide im Bereich hinter der Trennwand 13 nach oben und am Behälterkopf 5 aus dem Behälter 1 nach außen geführt und an jeweils eine Absaugpumpe 9 angeschlossen sind (dargestellt ist nur eine Pumpe 9).

Oberhalb der Aufwuchskörper 12 ist im Behälterinnenraum 3 wenigstens ein Schaumsieb 14 angeordnet, welches verhindert, dass durch den Gasaustritt 6 auch nicht gasförmige Partikel austreten können. Ferner ist im Bereich des Gasaustrittes 6 eine Stoffaustauschpackung 15 angeordnet.

Der Verfahrensablauf in der Vorrichtung ist wie folgt:
Nach einer mechanischen Vortrennung des Gärrestes, z.B. durch einen Separator, verbleibt eine Flüssigphase, welche etwa 80 % des Gesamtstoffstromes ausmacht. Diese Flüssigphase wird einem Flotationsverfahren unter Überdruck unterzogen, beispielsweise in einer Vorrichtung gemäß EP 1 294 475 B2, welches dafür sorgt, dass unter anderem feinste biologische Partikel durch die besonderen physikalischen Scher- und Gravitationskräfte aufgeschlossen werden, um die noch vorhandenen schnell verfügbaren biologischen Kohlenstoffe anschließend im Behälter 1 der Vorrichtung gemeinsam mit den noch verfügbaren Säuren über eine Verweildauer von vorzugsweise 12 Stunden weitestgehend abzubauen. Zudem sorgt das vorgeschaltete Flotationsverfahren für eine Phasentrennung des zugeführten Materials.

Bei der Einleitung der Flüssigphase durch den Zulauf 4 und die Beregnungseinrichtung 16 in den nicht unter Überdruck stehenden Behälter 1 findet eine Entspannung der Flüssigphase statt, welche zu einer schnellen Entgasung im Behälter 1 führt.

Für den schnellen Abbau der noch vorhandenen schnell verfügbaren biologischen Kohlenstoffe wird der Inhalt des Behälters 1, also der Gärrest, auf vorzugsweise 52,5° erhitzt. Außerdem sind dem Behälter 1 die vorerwähnten Aufwuchskörper 12 zugegeben, welche als Siedlungsfläche den methanbildenden Mikroorganismen dienen. Für eine weitere Steigerung der Prozessabläufe können geeignete Additive, Makroelemente und biologisch verfügbare Spurenelemente in den Behälter hinzugefügt werden.

Die während der Verweildauer der flüssigen Gärreste im Behälterinnenraum 3 entstehenden bzw. freigesetzten Gase werden kontinuierlich oder diskontinuierlich durch den Gasaustritt 6 abgeführt und können z.B. energetisch genutzt werden. Am Ende einer durchschnittlichen Verweildauer von ca. 12 Stunden wird der Großteil der gereinigten Flüssigkeit durch den Auslass 10 aus dem Behälter 1 abgeführt und z.B. einem Endlager zugeführt. Das Verfahren läuft dabei bevorzugt kontinuierlich, kann aber alternativ auch im Batchbetrieb erfolgen. Anders konzentrierte Flüssigphasen können gewünschtenfalls über weitere Entnahmeanschlüsse 11 abgezogen werden. Im Bodenbereich des Behälters abgesetzte Festpartikel können als Bioschlamm aus dem Austritt 8 abgepumpt werden.

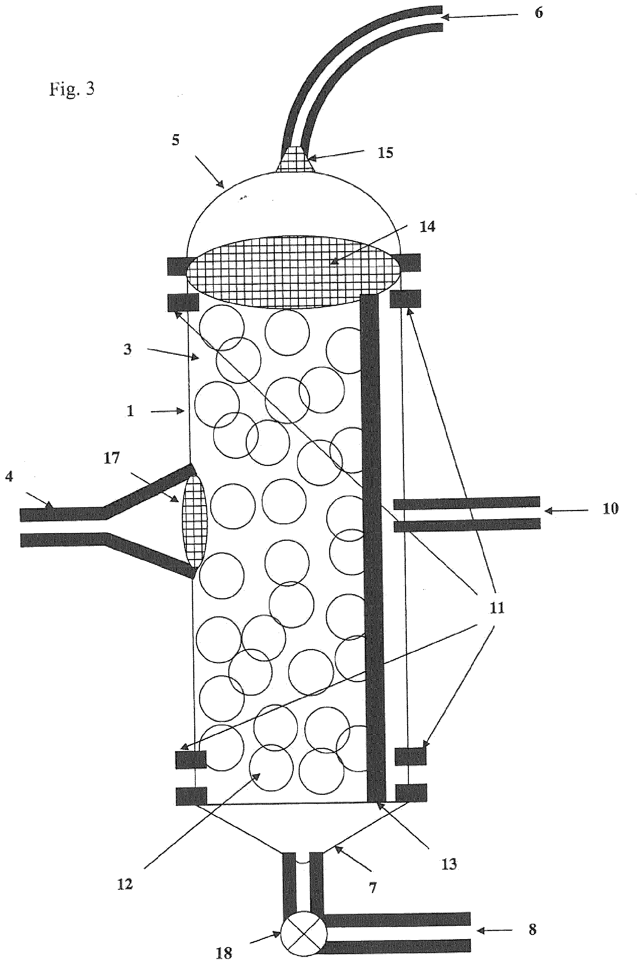

In den Figuren 3 und 4 ist ein abgewandeltes Ausführungsbeispiel des erfindungsgemäßen Behälters 1 dargestellt, wobei in den Figuren, sofern gleiche Teile betroffen sind, dieselben Bezugszeichen wie in den Figuren 1 und 2 verwandt sind. Die Ausführungsform nach Figuren 3 und 4 eignet sich insbesondere für einen nicht doppelwandigen Behälter.

Im Unterschied zur Ausführungsform nach Figuren 1 und 2 weist der Behälter einen trichterartigen Zulauf 4 für die Flüssigphase auf. Dabei erweitert sich der Zulauf 4 zum Behälter 1 hin, was eine Entspannung der Flüssigphase zwecks Entgasung begünstigt. Der trichterartige Zulauf 4 ist in etwa im mittleren Höhenniveau des Behälters 1 seitlich angeordnet. Dabei ist im trichterartigen Zulauf 4 vorzugsweise ein Gitter 17 angeordnet, welches eine zusätzliche Verwirbelung bewirkt und verhindert, dass die Aufwuchskörper 12 in den Zulauf 4 gelangen könne. Dem Zulauf 4 ist vorzugsweise eine nicht dargestellte Flotationseinrichtung vorgeschaltet.

Der Bioschlammaustritt 8 ist im Behälterboden 7 angeordnet und über eine Absperreinrichtung 18 aus dem Behälter 1 geführt. Etwa im selben Höhenniveau wie der Zulauf 4 ist auf der dem Zulauf 4 abgewandten Seite des Behälters 1 hinter der Trennwand 13 der Austritt 10 für die aufbereitete Flüssigkeit angeordnet und seitlich aus dem Behälter 1 geführt. Über der Höhe des Behälters 1 können weitere Entnahmeanschlüsse 11 und/oder Schaugläser angeordnet sein.

Der Funktionsablauf in dem Behälter nach Figuren 3 und 4 ist ansonsten genauso wie bei der Ausführungsform nach Figuren 1 und 2.

### Bezugszeichenliste

- 1: Behälter
- 2: Heizung
- 3: Behälterinnenraum
- 4: Zulauf
- 5: Behälterkopf
- 6: Gasaustritt
- 7: Behälterboden
- 8: Bioschlammaustritt
- 8a: Austrittsleitung
- 9: Pumpe
- 10: Austritt
- 10a: Austrittsleitung
- 11: Entnahmeanschlüsse
- 12: Aufwuchskörper
- 13: Trennwand
- 14: Schaumsieb
- 15: Stoffaustauschpackung
- 16: Beregnungseinrichtung
- 17: Gitter
- 18: Absperreinrichtung

## Patentansprüche

1. Verfahren zur Aufbereitung von Gärresten aus Biogasanlagen, bei welchem die Flüssigphase der Gärreste einer Flotationseinrichtung zugeführt wird,
**dadurch gekennzeichnet,**
**dass** in der Flotationseinrichtung unter Überdruck feinste biologische Partikel aufgeschlossen werden und die aus der Flotationseinrichtung entnommene Flüssigphase mit den aufgeschlossenen Partikeln in einen temperierten Behälter (1) eingeleitet und entspannt wird und in diesem über eine Verweildauer von mehreren Stunden verbleibt, wobei der Behälter (1) über seiner Höhe mit Aufwuchskörpern (12) befüllt ist und wobei freigesetztes oder entstehendes Gas am Behälterkopf (5) abgeführt wird.

2. Verfahren nach Anspruch 1,
**dadurch gekennzeichnet,**
**dass** die Temperatur im Behälter (1) zwischen 35° und 60° C beträgt.

3. Verfahren nach Anspruch 1 oder 2,
**dadurch gekennzeichnet,**
**dass** die Verweildauer der Flüssigphase im Behälter (1) zwischen 2 und 48 Stunden beträgt.

4. Vorrichtung zur Durchführung des Verfahrens nach einem oder mehreren der Ansprüche 1 bis 3,
**gekennzeichnet durch**
eine Flotationseinrichtung und einen temperierten Behälter (1) mit einem mit der Flotationseinrichtung verbundenen Zulauf (4) für die Flüssigphase, einem Gasaustritt (6) am Behälterkopf (5), einem Bioschlammaustritt (8) am Behälterboden (7) und einem Austritt (10) für die Flüssigphase, wobei der Behälter (1) über seiner Höhe mit Aufwuchskörpern (12) befüllt ist.

5. Vorrichtung nach Anspruch 4,
**dadurch gekennzeichnet,**
**dass** zwischen dem Austritt (10) für die Flüssigphase und dem mit Aufwuchskörpern (12) befüllten Bereich des Behälters (1) eine für die Aufwuchskörper undurchlässige Trennwand (13) angeordnet ist.

6. Vorrichtung nach Anspruch 4 oder 5,
**dadurch gekennzeichnet,**
**dass** der Zulauf (4) im Bereich des Behälterkopfes (5) angeordnet ist und in einer Beregnungseinrichtung (16) mündet.

7. Vorrichtung nach Anspruch 4, 5 oder 6,
**dadurch gekennzeichnet,**
**dass** der Austritt (10) für die Flüssigphase und der Bioschlammaustritt (8) an Austrittsleitungen (10a, 8a) angeschlossen sind, welche am Behälterkopf (5) aus dem Behälter (19) nach außen geführt sind.

8. Vorrichtung nach einem oder mehreren der Ansprüche 4 bis 7,
**dadurch gekennzeichnet,**
**dass** oberhalb der Aufwuchskörper (12) im Behälter (1) wenigstens ein Schaumsieb (14) angeordnet ist.

9. Vorrichtung nach einem oder mehreren der Ansprüche 4 bis 8,
**dadurch gekennzeichnet,**
**dass** im Bereich des Gasaustrittes (6) eine Stoffaustauschpackung (15) angeordnet ist.

10. Vorrichtung nach einem oder mehreren der Ansprüche 4 bis 9,
**dadurch gekennzeichnet,**
**dass** der Behälter (1) wenigstens einen weiteren Entnahmeanschluss (11) aufweist.

## Claims

1. Method for processing fermentation residues from biogas plants, in which the liquid phase of the fermentation residues is fed to a flotation apparatus,
**characterised in that**
fine biological particles are broken down under excess pressure in the flotation apparatus and the liquid phase extracted from the flotation apparatus is introduced with the broken-down particles into a temperature-controlled container (1) and depressurised and remains in the latter for a dwell time of several hours, wherein the container (1) is filled up its height with growth bodies (12) and wherein released or emerging gas is discharged at the container head (5).

2. Method according to claim 1,
**characterised in that**
the temperature in the container (1) is between 35° and 60°C.

3. Method according to claim 1 or 2,
**characterised in that**
the dwell time of the liquid phase in the container (1) is between 2 and 48 hours.

4. Device for performing the method according to one or more of claims 1 to 3,
**characterised by**
a temperature-controlled container (1) with an inlet (4) connected to the flotation apparatus for the liquid phase, a gas outlet (6) at the container head (5), a biosludge outlet (8) at the container bottom (7) and an outlet (10) for the liquid phase, wherein the container (1) is filled up its height with growth bodies (12).

5. Device according to claim 4,
**characterised in that**
a partition (13) which is impermeable to the growth bodies is arranged between the outlet (10) for the liquid phase and the region of the container (1) filled with growth bodies (12).

6. Device according to claim 4 or 5,
**characterised in that**
the inlet (4) is arranged in the region of the container head (5) and opens into a sprinkling apparatus (16).

7. Device according to claim 4, 5 or 6,
**characterised in that**
the outlet (10) for the liquid phase and the biosludge outlet (8) are connected to outlet lines (10a, 8a) which are led to the outside of the container (19) at the container head (5).

8. Device according to one or more of claims 4 to 7,
**characterised in that**
at least one foam sieve (14) is arranged above the growth bodies (12) in the container (1).

9. Device according to one or more of claims 4 to 8,
**characterised in that**
a mass transfer packing (15) is arranged in the region of the gas outlet (6).

10. Device according to one or more of claims 4 to 9,
**characterised in that**
the container (1) has at least one further extraction connection (11).

## Revendications

1. Procédé de préparation de résidus de fermentation provenant d'installations de biogaz, pour lequel la phase liquide des résidus de fermentation est amenée à un dispositif de flottation,
**caractérisé en ce que**
dans le dispositif de flottation des particules biologiques les plus fines sous surpression sont désagrégées et la phase liquide prélevée du dispositif de flottation est introduite et détendue avec les particules désagrégées dans un récipient tempéré (1) et reste dans celui-ci pendant une durée de séjour de plusieurs heures, dans lequel le récipient (1) est rempli sur sa hauteur avec des corps de prolifération (12) et dans lequel du gaz dégagé ou se formant est évacué au niveau de la tête de récipient (5).

2. Procédé selon la revendication 1,
**caractérisé en ce que**
la température dans le récipient (1) s'élève entre 35° et 60 °C.

3. Procédé selon la revendication 1 ou 2,
**caractérisé en ce que**
la durée de séjour de la phase liquide dans le récipient (1) s'élève entre 2 et 48 heures.

4. Dispositif de réalisation du procédé selon l'une quelconque ou plusieurs des revendications 1 à 3,
**caractérisé par**
un dispositif de flottation et un récipient tempéré (1) avec une alimentation (4) reliée au dispositif de flottation pour la phase liquide, une sortie de gaz (6) au niveau de la tête de récipient (5), une sortie de boue biologique (8) au niveau du fond de récipient (7) et une sortie (10) pour la phase liquide, dans lequel le récipient (1) est rempli sur sa hauteur de corps de prolifération (12).

5. Dispositif selon la revendication 4,
**caractérisé en ce qu'**
entre la sortie (10) pour la phase liquide et la zone remplie de corps de prolifération (12) du récipient (1) une paroi de séparation (13) imperméable pour les corps de prolifération (13) est agencée.

6. Dispositif selon la revendication 4 ou 5,
**caractérisé en ce que**
l'alimentation (4) est agencée dans la zone de la tête de récipient (5) et débouche dans un dispositif d'arrosage (16).

7. Dispositif selon la revendication 4, 5 ou 6,
**caractérisé en ce que**
la sortie (10) pour la phase liquide et la sortie de boue biologique (8) sont raccordées aux conduites de sortie (10a, 8a) qui sont guidées vers l'extérieur au niveau de la tête de récipient (5) hors du récipient (19).

8. Dispositif selon l'une quelconque ou plusieurs des revendications 4 à 7,
**caractérisé en ce qu'**
au moins un tamis à mousse (14) est agencé au-dessus des corps de prolifération (12) dans le récipient (1).

9. Dispositif selon l'une quelconque ou plusieurs des revendications 4 à 8,
**caractérisé en ce qu'**
un emballage de transfert de matière (15) est agencé dans la zone de la sortie de gaz (6).

10. Dispositif selon l'une quelconque ou plusieurs des revendications 4 à 9,
**caractérisé en ce que**
le récipient (1) présente au moins un autre raccord de prélèvement (11).
